# EUROPEAN PATENT APPLICATION

(11) **EP 2 309 248 A2**
(43) Date of publication of application: **13.04.2011**
(21) Application number: 10466002.2
(22) Date of filing: 20.01.2010
(51) Int. Cl.: G01N 19/02, G01N 19/04, G01N 33/42

(54) **Mobile device for measuring the adhesion of a road surface**

(30) Priority: 06.10.2009 CZ 200921764 U
(71) Applicant: Centrum Dopravniho Vyzkumu, 63600 Brno (CZ)
(72) Inventor: Josef Andres, 62800 Brno (CZ)
(74) Representative: Kania, Frantisek

(57) **Abstract**

A mobile device for measuring the adhesion of a road surface is formed by a driving unit (1), to whose driving shaft (2) a measuring wheel (3) is attached, whose circular friction surface abuts the measured road, and it is fitted with a control unit (6) comprising a central processing unit (7) to whose inputs a sensor (9) for measuring the pressure of the measuring wheel (3) on the measured road and a speed sensor (8) are connected.

## Description

### Field of the invention

The invention relates to a mobile device for measuring the adhesion of a road surface.

### Background of the invention

At present, two methods are applied for evaluating the adhesion properties of roads and/or the roughness of their surfaces.

According to the first method, a certain amount of sand of exactly defined fineness ratio or grain size distribution is spread on the road and the size of the spread circular surface or cake is measured.

The drawback of this measurement method consists in its unsatisfactory accuracy.

The second method consists in measuring the road roughness by means of a pendulum, wherein its angle of swinging is measured. The friction surface of the pendulum is made of a rubber block of exactly defined properties.

The drawback of this measurement method consists in the fact that for the purpose of its accuracy, it is indispensable to check not only the shape of the rubber block, but also the properties of the material the block is made of, as they can considerably vary in time, for example due to the hardening of rubber. Another important factor that must be eliminated is the pull of gravity with respect to the inclined surface of the road, which is very seldom actually horizontal, i.e. perpendicular to the axis of the earth. Another drawback of this measurement method is its material and space exigency.

A common disadvantage of the two methods subsists in the fact that both of them are rather time-consuming. Neither of the described procedures is suitable for fast measurements that would render sufficient accuracy.

### Summary of the invention

The above drawbacks of the state-of-the art technology are to a great extent eliminated by the mobile device for measuring the adhesion of a road surface, wherein the invention essentially consists in that the device comprises a driving unit to whose driving shaft a measuring wheel with a circular friction surface abutting to the measured road is attached. The measuring device is also equipped with a control unit processing data from a pressure sensor for sensing the pressure of the measuring wheel on the road and data from a speed sensor.

Advantageous embodiments of this invention comprise the driving unit in the form of an electric motor, which can be powered by a portable accumulator.

According to another preferred embodiments of the invention, the driving unit is fitted on a support stand having adjustable length of the legs of a telescopic tripod, which eliminates - optionally in combination with a hinged arrangement of the measuring wheel on the driving shaft - various inclination angles of the measured surface with respect to the earth's axis of gravity.

Last but not least, the advantage of the invention consists in the fact that the measuring surface of the wheel can be made of an analogous type of rubber as rubber used in the manufacture of motor-car tyres.

### Brief description of the drawings

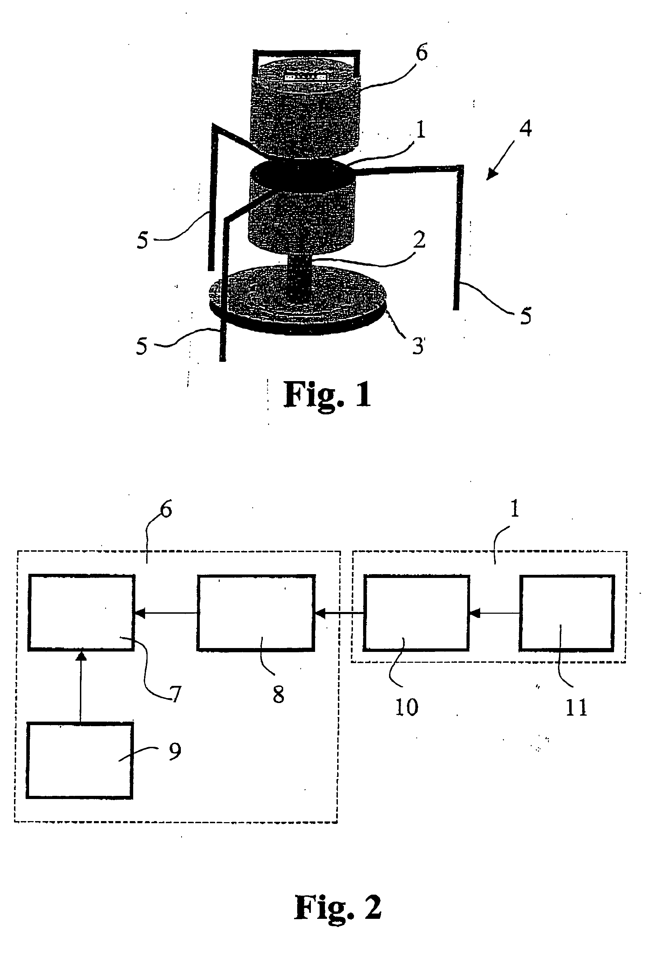

The invention is schematically represented in Fig. 1, while Fig. 2 is a schematic representation of the circuitry arrangement for controlling the mobile device for measuring the adhesion of a road surface according to the present invention.

### Detailed description of the exemplifying embodiments

Fig. 1 presents a mobile device for measuring the adhesion of a road surface according to the present invention, which consists of a driving unit **1**, to whose driving shaft **2** a measuring wheel **3** is attached, whose circular friction surface abuts the measured road. The mobile device for measuring the adhesion of the road surface is fixed in a support stand **4** having adjustable length of its legs **5**. The support stand **4** is a tripod having telescopic legs **5**. A control unit **6** is connected to the driving unit **1**.

Fig. 2 presents a preferred embodiment of the circuitry of the control unit **6**. The control unit **6** consists of a central processing unit **7**, to whose inputs a revolution counter **8** and a sensor **9** for measuring pressure of the measuring wheel **3** on the measured road are connected. The revolution counter **8** is connected to the output of the driving unit **1**, which consists of electric motor **10** powered by a portable accumulator.

In operation, the mobile device for measuring the adhesion of a road surface is set on the measured surface so that the circular friction surface of the measuring wheel **3** abuts the measured surface. This is effected by an appropriate setting of the telescopic legs **5** of the support stand **4**, optionally using an articulated joint (not shown). Then, the measuring wheel **3** is swung away from the road and the electric motor **10**, which is usually driven by an accumulator **11**, starts spinning the measuring wheel **3** up to the service speed. Having reached the service speed, the measuring wheel **3** is swung down to the road. Measuring the pressure of the measuring wheel **3** on the measured road, the revolution counter **8** measures the revolution run-out and/or the angular run-out. Then, the central processing unit **7** calculates the value of adhesion using the measured values, i.e. on the basis of the initial rotation speed, the rotation speed run-out and/or the angular run-out and on the basis of the value of pressure the measuring wheel **3** on the measured road.

Prior to starting the measurements, the mobile device for measuring the adhesion of a road surface is usually calibrated measuring the adhesion on a known surface of reference pads and subsequently rectified according to the measured values, so that the measurement may be accurate even in case of ageing of the friction surface of the measuring wheel.

### Industrial applicability

The mobile device for measuring the adhesion of a road surface may be used not only in construction or repairs of roads, but also in the detection of instantaneous adhesion values, e.g. during investigations of road accidents.

## Claims

1. A mobile device for measuring the adhesion of a road surface, **characterised in that** it is formed by a driving unit (1), to whose driving shaft (2) a measuring wheel (3) is attached, whose circular friction surface abuts the measured road, and it is also provided with a control unit (6) comprising a central processing unit (7) to whose inputs a sensor (9) for measuring the pressure of the measuring wheel (3) on the measured road and a speed sensor are connected.

2. The mobile device for measuring the adhesion of a road surface according to claim 1, **characterised in that** the driving unit (1) consists of an electric motor (10) connected with its supply input to an accumulator (11).

3. The mobile device for measuring the adhesion of a road surface according to claim 1, **characterised in that** the driving unit (1) is fixed on a support stand (4) having adjustable length of its legs (5).

4. The mobile device for measuring the adhesion of a road surface according to claim 3, **characterised in that** the support stand (4) having adjustable length of its legs (5) is designed as a tripod with telescopic legs (5).

5. The mobile device for measuring the adhesion of a road surface according to claim 1, **characterised in that** the measuring wheel (3) is attached to driving wheel (2) by means of an articulated joint.

6. The mobile device for measuring the adhesion of a road surface according to claim 1, **characterised in that** the measuring surface of measuring wheel (3) is made of rubber of the type used in the manufacture of car tyres.
